# EUROPÄISCHE PATENTSCHRIFT

(11) **EP 0 673 264 B1**
(45) Veröffentlichungstag und Bekanntmachung des Hinweises auf die Patenterteilung: **28.06.2000**
(21) Anmeldenummer: 94902716.3
(22) Anmeldetag: 08.12.1993
(51) Int. Cl.: A61M 15/00

(54) **INHALATIONSGERÄT**
INHALER
APPAREIL D'INHALATION

(30) Priorität: 11.12.1992 AT 245092
(43) Veröffentlichungstag der Anmeldung: 27.09.1995
(73) Patentinhaber: AstraZeneca AB, 151 85 Södertälje (SE)
(72) Erfinder: HATSCHEK, Rudolph, A., CH-1700 Fribourg (CH); HURKA, Wilhelm, A-9851 Lieserbrücke (AT)
(86) Internationale Anmeldenummer: EP9303453
(87) Internationale Veröffentlichungsnummer: WO9413348

(56) Entgegenhaltungen:
- EP-A- 0 215 559
- EP-A- 0 407 028
- EP-A- 0 516 510
- WO-A-91/12040
- WO-A-92/00771
- FR-A- 967 494
- US-A- 4 274 403

## Beschreibung

Die Erfindung betrifft ein Inhalationsgerät zur Dosierung und Verteilung von Festkörpern, insbesondere von pharmakologisch wirksamen Substanzen im festen Aggregatzustand, in die Atemluft, bestehend aus einem mit einem Lufteinlaß und einem Luftansaugöffnung dienenden Luftauslaß versehenen Körner, in dessen Inneren eine in sich geschlossene, zumindest angenähert kreisringförmige Kammer vorgesehen ist, an die tangential ein mit dem Lufteinlaß in Verbindung stehender Lufteinströmkanal anschließt und der ein mit dem Luftauslaß in Verbindung stehendes Luftausströmsystem zugeordnet ist, durch das die Luft aus der Ringkammer zentripetal ausströmen kann, und einer in der Ringkammer unverlierbar aufgenommenen und durch den Ansaugluftstrom in eine Umlaufbewegung versetzbaren Kugel, wobei der Körner aus einem Träger, in dem die Ringkammer, der Lufteinströmkanal und das Luftausströmsystem ausgespart sind, und aus einem den Lufteinlaß und den Luftauslaß aufweisenden Deckblatt besteht, wobei in dem Träger ein an sich bekanntes, eine für eine Vielzahl von Inhalationen ausreichende Menge an Festkörpern enthaltendes Reservoir ausgespart ist, und wobei in an sich bekannter Weise eine manuell betätigbare Dosiereinrichtung vorgesehen ist, mit der jeweils eine für eine Inhalation ausreichende Menge an Festkörpern aus dem Reservoir entnehmbar und in den Weg des Ansaugluftstromes transportierbar ist.

Bei dem bekannten Inhalationsgerät dieser Art (EP-B-0 215 559), das für den einmaligen Gebrauch bestimmt ist, sind die zu dosierenden und zu verteilenden Festkörper zwischen der Kugel und der Ringkammeroberfläche verteilt, insbesondere indem sie in Form eines Films auf die Kugel- und/oder Ringkammeroberfläche aufgebracht sind. Durch die durch den Ansaugluftstrom bewirkte Umlaufbewegung der Kugel entsteht ein Spektrum von Relativbewegungen zwischen der feststehenden Ringkammeroberfläche und der in allen Raumrichtungen rotierenden Kugel, welche Relativbewegungen auf die zwischen der Kugel- und Ringkammeroberfläche verteilten Festkörper Kompressions- und Scherkräfte ausüben, die zu einem Zerkleinerungsprozeß ähnlich wie in einer Kugelmühle führen. Die so erzeugten Festkörperpartikel (das Mahlgut) werden dabei laufend einem Aerosolbildungsprozeß unterworfen, indem sie in dem Ringkammer durch das Ausströmsystem zentripetal verlassende Luft verteilt werden. Das Wirkprinzip dieses bekannten Geräts ist somit eine Kombination aus mechanischem Wirkprinzip (Kugelmühle) und aerodynamischem Wirkprinzip (Aerosolbildung). Der Vorteil dieses bekannten Geräts liegt vor allem darin, daß es einfach und kostengünstig und in sehr kleiner Größe (Taschenformat) herstellbar ist.

Ein nicht zum bekannten Stand der Technik zählender Vorschlag geht dahin, bei gewissen Anwendungen die Kugel wegzulassen und die Festkörper in der Ringkammer bereits in der für die Applikation geeigneten feinpulvrigen Form (Korngrößenverteilung) und in der für die einmalige Dosierung notwendigen Menge lose vorzusehen. Bei einen solchen Gerät werden die Festkörper ohne mechanisches Hilfsmittel ausschließlich durch den Ansaugluftstrom in die Atemluft verteilt. Der Vorteil eines solchen Geräts wird vor allem in der durch den Wegfall der Kugel erzielten Einsparung von Material- und Arbeitskosten und in einer vereinfachten Einbringung der Wirksubstanz in das Gerät gesehen.

Nachteilig an nur für einen einmaligen Gebrauch bestimmten Geräten ist zum einen, daß der z.B. an Asthma leidende Patient sicherheitshalber immer mehrere Geräte mit sich führen muß, und zum anderen, daß die Entsorgung von Wegwerfartikeln ein immer größer werdendes Problem darstellt.

Aufgabe der vorliegenden Erfindung ist daher, ein Inhalationsgerät zu schaffen, das ebenso wie die oben beschriebenen Geräte in sehr kleiner Größe realiserbar ist, das aber eine Vielzahl von Inhalationen ermöglicht.

Diese Aufgabe wird ausgehend von einem Inahaltionsgerät der eingangs geschilderten Art erfindungsgemäß dadurch gelöst, daß die Dosiereinrichtung als ein in einer Bohrung des Trägers zwischen zwei Arbeitsstellungen verschiebbarer Dosierkolben ausgebildet ist, wobei der Dosierkolben über eine Dosierkammer verfügt, die in der einen Arbeitsstellung des Dosierkolbens mit einer Ausgabeöffnung des Reservoirs und in der anderen Arbeitsstellung mit dem Lufteinströmkanal in Verbindung steht.

Es sind zwar bereits Inhalationsgeräte bekannt, die nur über den Atemluftstrom des Benützers betreibbar sind und auch über ein Reservoir für eine für mehrere Inhalationen ausreichende Menge an Festkörpern sowie eine manuell betätigbare Dosiereinrichtung für die Entnahme einer für eine Inhalation ausreichenden Festkörpermenge aus dem Reservoir und den Transport derselben in den Weg des Ansaugluftstromes verfügen, jedoch sind diese Geräte aufgrund ihres Aufbaus nicht in so kleiner Größe und auch nicht so billig herstellbar wie das erfindungsgemäße Gerät. Auch von ihrer Form her sind diese Geräte schlecht dafür geeignet, vom Patienten immer mitgeführt zu werden. Demgegenüber kann das erfindungsgemäße Gerät in sehr flacher Form hergestellt werden und ist damit leicht in jeder Tasche unterzubringen.

In der bevorzugten Ausführungsform ist die manuell betätigbare Dosiereinrichtung als ein in einer Bohrung des Trägers zwischen zwei Arbeitsstellungen verschiebbarer Dosierkolben ausgebildet, der über eine Dosierkammer verfügt, die in der einen Arbeitsstellung des Dosierkolbens mit einer Ausgabeöffnung des Reservoirs und in der anderen Arbeitsstellung mit dem Lufteinströmkanal in Verbinddung steht.

Ein solches Inhalationsgerät kann bei etwa gleicher Größe wie das Gerät nach der genannten EP-B-0 215 559 etwa 200 bis 250 Inhalationen ermöglichen.

Nach einem weiteren Merkmal der Erfindung kann durch hermetische Abdichtung des Dosierkolbens der den Lufteinlaß, den Lufteinströmkanal, die Ringkammer, das Luftausströmsystem und den Luftauslaß umfassende Zerstäuberteil des Geräts vom Reservoirteil getrennt sein. Dadurch ist eine allfällige Reinigung des Zerstäuberteils von Resten an Festkörpern oder Verunreinigungen möglich.

Zweckmäßig kann ferner im Deckblatt über der Ausgabeöffnung des Reservoirs eine Lupe vorgesehen sein. Dies ermöglicht eine visuelle Kontrolle der Füllung der Dosierkammer des Dosierkolbens.

Schließlich ist es vorteilhaft, wenn der vom Träger gebildete Boden und der vom Deckblatt gebildete Deckel des Reservoirs als Resonanzböden ausgebildet sind, die durch die umlaufende Kugel entstehende Vibration übernehmen, da dies bei jeder Inhalation zu einer Auflockerung der im Reservoir enthaltenen Festkörper führt.

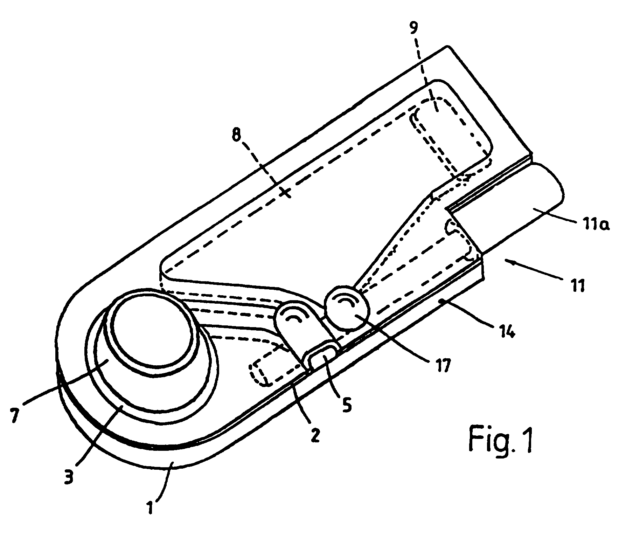

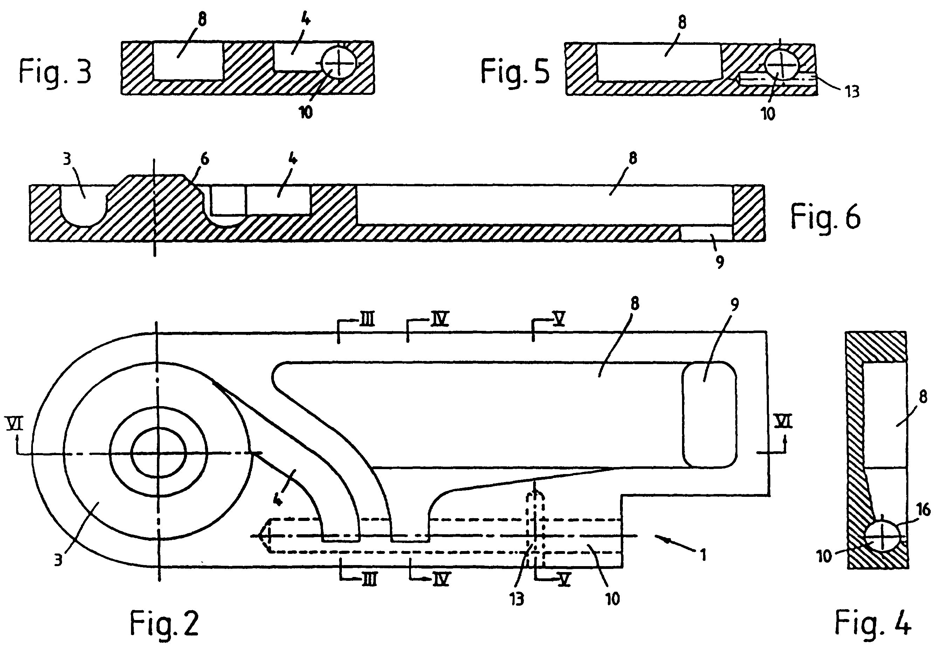

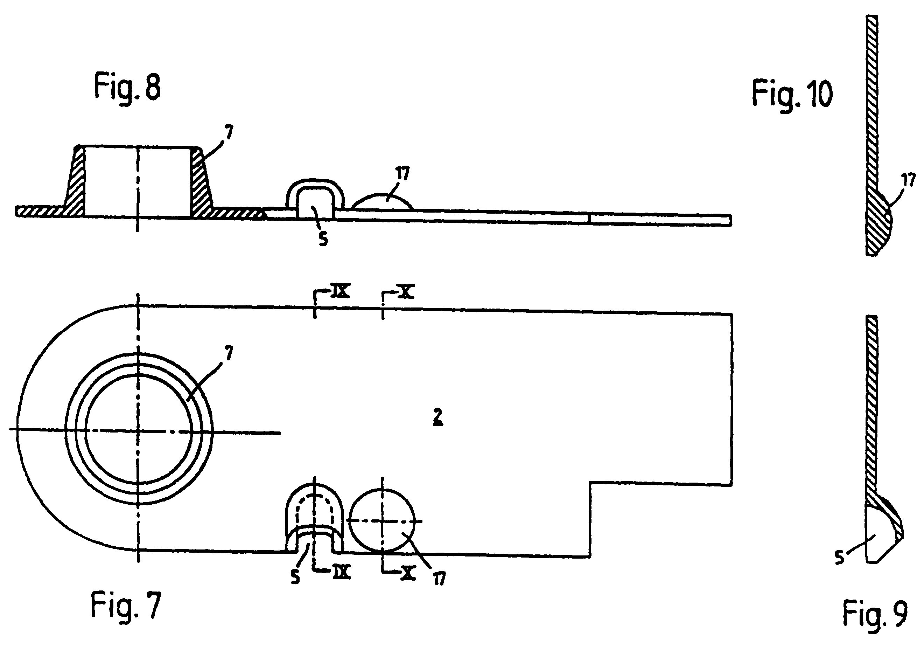

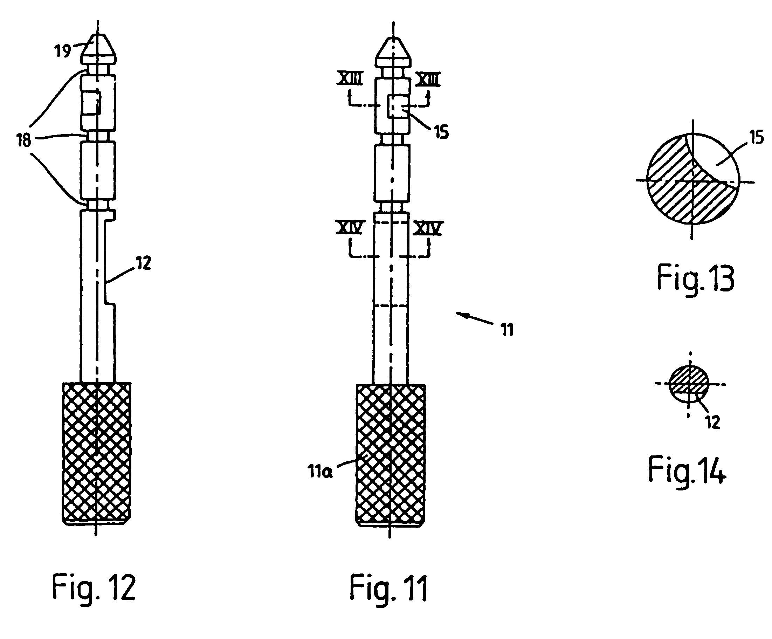

Nachstehend ist die Erfindung anhand eines in den beigeschlossenen Zeichnungen gezeigten Ausführungsbeispieles näher erläutert. In den Zeichnungen zeigen: Figur 1 eine isometrische Darstellung eines erfindungsgemäßen Inhalationsgeräts, Figur 2 eine Draufsicht des einen ersten Teil des Körpers des Geräts bildenden Trägers, Figur 3 einen Schnitt nach der Linie III-III in Figur 2, Figur 4 einen Schnitt nach der Linie IV-IV in Figur 2, Figur 5 einen Schnitt nach der Linie V-V in Figur 2, Figur 6 einen Schnitt nach der Linie VI-VI in Figur 2, Figur 7 eine Draufsicht auf das einen zweiten Teil des Körpers bildende Deckblatt, Figur 8 das Deckblatt in Vorderansicht, teilweise im Schnitt, Figur 9 einen Schnitt nach der Linie IX-IX in Figur 7, Figur 10 einen Schnitt nach der Linie X-X in Figur 7, Figur 11 die als Dosierkolben ausgebildete Dosiereinrichtung des Geräts in Draufsicht, Figur 12 den Dosierkolben in Seitenansicht, Figur 13 in vergrößten Maßstab einen Schnitt nach der Linie XIII-XIII in Figur 11 und Figur 14 einen Schnitt nach der Linie XIV-XIV in Figur 11.

Das in Figur 1 dargestellte Inhalationsgerät weist, wie aus der obengenannten EP-B-0 215 559 bekannt, einen aus einem Träger 1 und einem Deckblatt 2 bestehenden Körper auf, wobei der Träger 1, der in den Figur 2 bis 6 näher dargestellt ist, eine Materialaussparung besitzt, die im Inneren des Körpers eine kreisringförmige Kammer 3, einen an diese Ringkammer 3 tangential anschließenden Lufteinströmkanal 4, der mit einem den Lufteinlaß des Gerätes bildenden Lufteintrittekanal 5 im Deckblatt 2 (Figur 7 bis 10) in Verbindung steht, und ein Luftausströmsystem 6 bildet, das mit einem in dem Deckblatt 2 ausgebildeten und den Luftauslaß des Gerätes bildenden Mundstück 7 in Verbindung ist und durch das die Luft in bezug auf die Ringkammer 3 zentripetal ausströmen kann.

In der Ringkammer 3 ist, wie aus der genannten EP-B-0 215 559 bekannt, eine (nicht dargestellte) Kugel unverifierbar angeordnet, die durch den beim Inhalieren durch das Mundstück 7 erzeugten Ansaugluftstrom in eine Umlaufbewegung innerhalb der Ringkammer versetzbar ist.

Eine weitere Materialaussparung im Träger 1 bildet ein Reservoir 8, in den eine für eine Vielzahl von Inhalationen, z.B. für 200 bis 250 Inhalationen, ausreichende Menge an Wirksubstanz aufnehmbar ist. Die Wirksubstanz kann in Form eines granulierten Pulvers, in Form von Mikrosphären oder in Form von Liposomen vorliegen. Die Füllung des Reservoirs 8 bei der Herstellung des Geräts erfolgt durch eine Einfüllöffnung 9 im Boden des Träges 1, die nach erfolgter Füllung durch ein nicht gezeigtes Blattmaterial, insbesondere ein Aluminiumblatt, verschlossen wird, das z.B. durch Heißsiegeln mit der Unterseite des Geräts fest verbunden wird. Dieses Aluminiumblatt kann gleichzeitig als Etikette mit Bedienungsanleitung dienen. Wenn erforderlich, kann auf der Innenseite des Aluminumblattes im Bereich der Einfüllöffnung 9 eine mit Silicagel gefüllte Trockenpatrone, die die Form der Einfüllöffnung hat, befestigt sein.

Zur Entnahme der für eine Inhalation notwendigen Wirksubstanzmenge aus dem Reservoir 8 und zum Führen derselben in den Weg des Ansaugluftstromes ist innerhalb einer Bohrung 10 im Träger 1 ein Dosierkolben 11 (Figur 11 bis 14) manuell verschiebbar angeordnet. Zur manuellen Betätigung dieses Dosierkolbens 11 dient ein Handgriff 11a. Durch einen mit einer gefrästen seitlichen Abflachung 12 des Dosierkolbens 11 zusammenwirkenden, in eine Bohrung 13 des Trägers 1 eingesetzen Stift 14 (Fig. 1) wird der Hub des Dosierkolbens 11 zwischen zwei definierten Arbeitsstellungen begrenzt und außerdem der Dosierkolben 11 gegen Verdrehung gesichert.

In der zurückgezogenen Arbeitsstellung des Dosierkolbens 11 steht eine durch eine gefräste Einsenkung mit konkavem Boden gebildete Dosierkammer 15 des Dosierkolbens 11 mit einer in die Bohrung 10 mündenden Ausgabeöffnung 16 (Figur 4) des Reservoirs 8 in Verbindung. In dieser Stellung des Dosierkolbens 11 wird die Dosierkammer 15 mit Wirksubstanz aus den Reservoir 8 durch Gravitation gefüllt, wobei dieser Vorgang durch Klopfen mit den Finger oder Schütteln vervollständigt werden kann. Zur visuellen Kontrolle dieses Füllvorganges ist am Deckblatt 1, bei dem es sich um einen Preßteil aus glasklarem Kunststoff handelt, über der Ausgabeöffnung 16 eine Lupe 17 ausgebildet. Die Dosiergenauigkeit hängt von der so kontrollierten Füllung ab, jedoch ergibt sich aus diesem Prinzip, daß eine Überdosierung ausgeschlossen ist.

Nach erfolgtem Füllvorgang wird der Dosierkolben 11 mittels seines Handgriffes 11a in die zweite, vorgeschobene Arbeitsstellung gebracht, inder die Dosierkammer 15 mit dem Lufteinströmkanal 4 in Verbindung steht, womit die in der Dosierkammer 15 vorhandene Wirksubstanze sich im Weg des Ansaugluftstromes befindet und das Gerät für eine Inhalation bereitsteht.

Durch das beim Inhalieren erfolgende Ansaugen von Luft an dem Mundstück 7, das sich sowohl zur Mund- als auch zur Naseninhalation eignet, tritt Luft durch den Lufteinlaß 5 ein, gelangt unter Mitnahme der Wirksubstanz durch den Lufteinströmkanal 4 in die Ringkammer 3 und treibt die darin befindliche Kugel an. Durch die Kugelbewegung wird die Wirksubstanz mechanisch aufbereitet und in die die Ringkammer 3 durch das Ausströmsystem 6 in Richtung Mundstück 7 verlassende Luft verteilt. Das durch die umlaufende Kugel erzeugte Geräusch ist übrigens für den Patienten ein akustisches Signal dafür, daß das Gerät ordnungsgemäß arbeitet. Zweckmäßig sind der Boden des Reservoirs bildende Deckblatt 2 so ausgeführt, daß sie die durch die umlaufende Kugel entstehende Vibration übernehmen (Resonanzboden), was bei jeder Inhalation zu einer Auflockerung der im Reservoir 8 enthaltenen Wirksubstanz führt.

Der Dosierkolben 11 weist drei Ringnuten 18 zur Aufnahme dreier (nicht dargestellter) O-Ringe auf, die für eine hermetische Abdichtung gegenüber der Wand der Bohrung 10 des Trägers 1 sorgen. Ein vorderer Kegelansatz 19 des Dosierkolbens 11 erleichtert das Aufbringen der drei O-Ringe. Dadurch, daß durch die hermetische Abdichtung des Dosierkolbens der den Lufteinlaß 5, den Lufteinströmkanal 4, die Ringkammer 3, das Luftausströmsystem 6 und den Luftauslaß 7 umfassende Zerstäuberteil von dem sogenannten Reservoirteil des Geräts getrennt ist, kann der Zerstäuberteil, wenn nötig, von Zeit zu Zeit z.B. mit Wasser gereinigt werden, um Reste an Wirksubstanz sowie Verunreinigungen zu entfernen.

Dadurch, daß das Deckblatt 2 transparent ist, hat man eine Kontrolle darüber, wieviel Wirksubstanz sich noch in dem Reservoir 8 befindet. Sollte es aus irgenwelchen Gründen notwendig sein, die Innenflächen des Reservoirs 8 mit einer Schutzschicht zu überziehen, muß wenigstens Vorsorge dafür getroffen werden, daß jener kleine Bereich transparent bleibt, der durch die Lupe 16 zur Kontrolle des Füllvorganges dient.

Das Deckblatt 2 ist mit dem z.B. aus ABS bestehenden Träger 1 über seine ganze Berührungsfläche hermetisch verbunden. Dies kann durch Heißsiegeln oder ein anderes Flächenbonding-Verfahren, wie z.B. Ultraschallschweißen, Anodic bonding oder Kleben, bewerkstelligt sein.

Es versteht sich, daß die Erfindung nicht auf das beschriebene und dargestellte Ausführungsbeispiel beschränkt ist.

Weiters könnte die Wirksubstanz, insbesondere wenn ein Schutz vor Luft und/oder Feuchtigkeit erforderlich ist-vordosiert in kleinen Kugeln mit einer berstbaren Hülle mit der erforderlichen Schutzeigenschaften vorliegen. In einem solchen Fall ist die Dosiereinrichtung zweckmäßig als ein den Erfordernissen angepaßter Einzelkugel-Transportkolben ausgebildet, durch den in der Endstellung auch die Zertrümmerung der Schutzhülle erfolgt. Die frei werdende Kugelfüllung wird dann wie bei der beschriebenen Ausführungsform in der Ringkammer weiterverarbeitet. Der Wirkstoff kann sowohl in sofort wirkender als auch in retardiert wirkender Form vorliegen, und es ist auch die Dosierung von klebrigen Substanzen, wie z.B. Peptiden, möglich. Die Kugeln werden in dem Reservoir zweckmäßig in einer einzigen Lage aufbewahrt. Bei einem Durchmesser der Kugel von etwa 1 bis 2 mm beträgt die Reservoirtiefe 1,3 bis 2,3 mm.

Schließlich muß die Ringkammer 3 nicht, wie dargestellt, exakt kreisförmig sein, sondern kann auch geringfügig elleptisch sein oder auf andere Weise etwas von der Kreisform abweichen.

## Patentansprüche

1. Inhalationsgerät zur Dosierung und Verteilung von Festkörpern, insbesondere von pharmakologisch wirksamen Substanzen im festen Aggregatzustand, in die Atemluft, bestehend aus einem mit einem Lufteinlaß (5) und einem Luftansaugöffnung dienenden Luftauslaß (7) versehenen Körper, in dessen Inneren eine in sich geschlossene, zumindest angenähert kreisringförmige Kammer (3) vorgesehen ist, an die tangential ein mit dem Lufteinlaß (5) in Verbindung stehender Lufteinströmkanal (4) anschließt und der ein mit dem Luftauslaß (7) in Verbindung stehendes Luftausströmsystem (6) zugeordnet ist, durch das die Luft aus der Ringkammer (3) zentripetal ausströmen kann, und einer in der Ringkammer (3) unverlierbar aufgenommenen und durch den Ansaugluftstrom in eine Umlaufbewegung versetzbaren Kugel, wobei der Körner aus einem Träger (1), in dem die Ringkammer (3), der Lufteinströmkanal (4) und das Luftausströmsystem (6) ausgespart sind, und aus einem den Lufteinlaß (5) und den Luftauslaß (7) aufweisenden Deckblatt (2) besteht, wobei in dem Träger (1) ein an sich bekanntes, eine für eine Vielzahl von Inhalationen ausreichende Menge an Festkörpern enthaltendes Reservoir (8) ausgespart ist, und wobei in an sich bekannter Weise eine manuell betätigbare Dosiereinrichtung (11) vorgesehen ist, mit der jeweils eine für eine Inhalation ausreichende Menge an Festkörpern aus dem Reservoir (8) entnehmbar und in den Weg des Ansaugluftstromes transportierbar ist, **dadurch gekennzeichnet**, daß die Dosiereinrichtung als ein in einer Bohrung (10) des Trägers (1) zwischen zwei Arbeitsstellungen verschiebbarer Dosierkolben (11) ausgebildet ist, wobei der Dosierkolben über eine Dosierkammer (15) verfügt, die in der einen Arbeitsstellung des Dosierkolbens mit einer Ausgabeöffnung (16) des Reservoirs (8) und in der anderen Arbeitsstellung mit dem Lufteinströmkanal (4) in Verbindung steht.

2. Inhalationsgerät nach Anspruch 1, dadurch gekennzeichnet, daß durch hermetische Abdichtung des Dosierkolbens (11) der den Lufteinlaß (5), den Lufteinströmkanal (4), die Ringkammer (3) das Luftausströmsystem (6) und den Luftauslaß (7) umfassende Zerstäuberteil des Geräts vom Reservoirteil getrennt ist.

3. Inhalationsgerät nach einem der Ansprüche 1 bis 2, dadurch gekennzeichnet, daß im Boden des Trägers (1) eine Einfüllöffnung (9) für das Reservoir (8) vorgesehen ist, die durch ein an der Unterseite des Trägers (1) befestigtes Blattmaterial, vorzugsweise ein Aluminiumblatt, verschlossen ist.

4. Inhalationsgerät nach einem der Ansprüche 1 bis 3, dadurch gekennzeichnet, daß im Deckblatt (2) über der Ausgabeöffnung (16) des Reservoirs (8) eine Lupe (17) vorgesehen ist.

5. Inhalationsgerät nach einem der Ansprüche 1 bis 4, dadurch gekennzeichnet, daß der vom Träger (1) gebildete Boden und der vom Deckblatt (2) gebildete Deckel des Reservoirs (8) als Resonanzböden ausgebildet sind, die die durch die umlaufende Kugel entstehende Vibration übernehmen.

## Claims

1. Inhalation device for dosing and dispersing solids, in particular pharmacologically active substances in the solid aggregate state, into the respiratory air, consisting of a body provided with an air inlet (5) and an air outlet (7) which serves as an air suction opening, in the inside of which body there is provided a self-contained, at least approximately circular chamber (3) which is adjoined tangentially by an air admission channel(4) communicating with the air inlet(5) and which is allocated an air delivery system (6) communicating with the air outlet(7), through which air delivery system (6) the air can flow out centripetally from the annular chamber (3), and a ball which is held trapped in the annular chamber (3) and which can be set in a rotational movement by the suction air stream, the body consisting of a support (1) in which the annular chamber (3), the air admission channel (4) and the air delivery system (6) are hollowed out, and of a cover plate (2) which includes the air inlet (5) and the air outlet (7), a reservoir (8) which is known per se and which contains a sufficient quantity of solids for a multiplicity of inhalations being hollowed out in the support (1), and a manually activatable dosing unit (11) being provided in a manner known per se, with which in each case a quantity of solids sufficient for one inhalation can be removed from the reservoir (8) and conveyed into the path of the suction air stream, characterized in that the dosing unit is designed as a dosing piston (11) which can be displaced between two working positions in a bore (10) of the support (1), the dosing piston having a dosing chamber (15) communicating, in the one working position of the dosing piston, with a discharge opening (16) of the reservoir (8), and, in the other working position, communicating with the air admission channel (4).

2. Inhalation device according to Claim 1, characterized in that the sprayer part of the device comprising the air inlet (5), the air admission channel (4), the annular chamber (3), the air delivery system (6) and the air outlet (7) is separated from the reservoir part by means of hermetic sealing of the dosing piston (11).

3. Inhalation device according to one of Claims 1 to 2, characterized in that a filling hole (9) for the reservoir (8) is provided in the base of the support (1), which filling hole (9) is closed by a sheet of material, preferably an aluminium sheet, secured on the underside of the support (1).

4. Inhalation device according to one of Claims 1 to 3, characterized in that a magnifying glass (17) is provided in the cover plate (2) above the discharge opening (16) of the reservoir (8).

5. Inhalation device according to one of Claims 1 to 4, characterized in that the base formed by the support (1) and the cover of the reservoir (8) formed by the cover plate (2) are designed as soundboards, which take up the vibration caused by the rotating ball.

## Revendications

1. Inhalateur pour le dosage et la dispersion de solides, en particulier de substances actives sur le plan pharmacologique à l'état solide, dans l'air inhalé, constitué d'un corps pourvu d'une entrée d'air (5) et d'une sortie d'air (7) servant d'ouverture d'aspiration d'air, à l'intérieur duquel une chambre (3) en forme d'anneau au moins approximativement circulaire, y incluse, est prévue, à laquelle est raccordé de manière tangentielle un conduit d'arrivée d'air (4) relié à l'entrée d'air (5) et à laquelle est adjoint un système d'écoulement d'air (6) relié à la sortie d'air (7), par lequel l'air peut s'écouler de manière centripète de la chambre annulaire (3), et d'une bille logée de manière imperdable dans la chambre annulaire (3) et pouvant être déplacée suivant un mouvement rotatif par le flux d'air aspiré, le corps étant constitué d'un support (1), par évidement duquel la chambre annulaire (3), le conduit d'arrivée d'air (4) et le système d'écoulement d'air (6) sont obtenus, et d'une feuille de couverture (2) présentant l'entrée d'air (5) et la sortie d'air (7), un réservoir (8) connu en soi, contenant une quantité de solides suffisante pour une pluralité d'inhalations, étant obtenu par évidement du support, et un dispositif de dosage (11) à commande manuelle étant prévu de manière connue en soi, avec lequel la quantité de solides suffisante pour une inhalation peut chaque fois être prélevée du réservoir (8) et être amenée sur le trajet du flux d'air aspiré, caractérisé en ce que le dispositif de dosage est réalisé sous la forme d'un piston doseur (11) pouvant coulisser dans un alésage (10) du support (1) entre deux positions de travail, le piston doseur disposant d'une chambre de dosage (15) qui est reliée, dans l'une des positions de travail, a l'orifice de sortie (16) du réservoir (8) et, dans l'autre position de travail, au conduit d'arrivée d'air (4).

2. Inhalateur suivant la revendication 1, caractérisé en ce que la partie atomiseur de l'appareil comprenant l'entrée d'air (5), le conduit d'arrivée d'air (4), la chambre annulaire (3), le système d'écoulement d'air (6) et la sortie d'air (7) est séparée de la partie réservoir par un joint hermétique du piston doseur (11).

3. Inhalateur suivant l'une quelconque des revendications 1 à 2, caractérisé en ce que, au fond du support (1), une ouverture de remplissage (9) du réservoir (8) est prévue qui est fermée par un matériau en feuille fixé sur le dessous du support (1), de préférence une feuille d'aluminium.

4. Inhalateur suivant l'une quelconque des revendications 1 à 3, caractérisé en ce qu'une loupe (17) est prévue dans la feuille de couverture (2) au-dessus de l'orifice de sortie (16) du réservoir (8).

5. Inhalateur suivant l'une quelconque des revendications 1 à 4, caractérisé en ce que le fond formé par le support (1) et le couvercle formé par la feuille de couverture (2) du réservoir (8) sont réalisés sous la forme de sources de résonance qui reprennent les vibrations engendrées par le mouvement rotatif de la bille.
